# EUROPEAN PATENT APPLICATION

(11) **EP 4 488 683 A1**
(43) Date of publication of application: **08.01.2025**
(21) Application number: 23759626.7
(22) Date of filing: 02.02.2023
(51) Int. Cl.: G01N 33/543, G01N 33/53

(54) **IMMUNOCHROMATOGRAPHIC TEST KIT**

(30) Priority: 28.02.2022 JP 2022030380
(71) Applicant: FUJIFILM Corporation, Tokyo 106-8620 (JP)
(72) Inventor: TAKEI, Toshiki, Ashigarakami-gun, Kanagawa 258-8538 (JP); SAITO, Koichi, Ashigarakami-gun, Kanagawa 258-8538 (JP)
(74) Representative: Dehns Germany Partnerschaft mbB
(86) International application number: PCT/JP2023/003479
(87) International publication number: WO 2023/162620

(57) **Abstract**

There is provided an immunochromatographic kit that makes it possible to suppress erroneous determination due to a shortage of an amount of a specimen at the time of collecting the specimen.

The immunochromatographic kit includes a cartridge that accommodates an examination strip on which a specimen is spread and an examination region for capturing a test substance contained in the specimen is provided, and an accessory that is used for carrying out at least any of collection of the specimen or pretreatment carried out on the specimen before the specimen is spread on the examination strip, where a part of at least any of the examination strip or the accessory includes a portion in which a coloring reaction is exhibited by a reaction with a specimen-derived component different from the test substance in the specimen.

## Description

### BACKGROUND OF THE INVENTION

### 1. Field of the Invention

The present disclosure relates to an immunochromatographic kit.

### 2. Description of the Related Art

As a method of determining whether or not a specimen such as a biological specimen contains a test substance, an immunological determination method is generally used. Among the above, an immunochromatographic method is highly convenient since the operation is easy and the measurement can be carried out in a short time.

In the immunochromatographic method, an examination strip, on which an antibody (a capture antibody) that specifically binds to a test substance (for example, an antigen) is immobilized in an examination region, is used (see JP2015-105858A, JP2018-522221A, and the like). A specimen is spread on the examination strip, and then the presence or absence of the test substance can be determined by whether or not a line is developed in the examination region. A cartridge encompassing such an examination strip has been put into practical use, and accessories such as a specimen collecting tool for collecting a nasopharyngeal swab fluid or a nasal cavity swab fluid as a specimen and an extraction solution for extracting a test substance from a specimen are provided as an immunochromatographic kit.

Such an immunochromatographic kit is used, for example, for an examination for determining whether or not a subject is affected with an infectious disease due to infection with the influenza virus or novel coronavirus (COVID-19). In particular, it is useful for point of care testing (POCT) which is carried out in a medical field, a self-test in which a patient himself/herself examinations whether or not he/she is affected with an infectious disease at home, or the like.

### SUMMARY OF THE INVENTION

In general, in a case where a specimen is a colorless and transparent specimen, such as a nasopharyngeal swab fluid or a nasal cavity swab fluid, it is difficult to determine whether or not the collection of the amount of the specimen necessary for the examination has been successful. The shortage of the amount of the specimen has a large influence on the examination accuracy. In particular, in a case where a non-medical worker who is not familiar with the collection of a specimen collects a specimen, for example, in a case of a self-test in which a patient himself/herself carries out an examination at home, there is a high possibility that an amount of a specimen is insufficient. Therefore, a measure for preventing erroneous determination due to a shortage of an amount of a specimen at the time of collecting the specimen has been desired.

The present disclosure has been made in consideration of the above circumstances, and an object of the present disclosure is to provide an immunochromatographic kit that makes it possible to suppress erroneous determination due to a shortage of an amount of a specimen at the time of collecting the specimen.

An immunochromatographic kit according to the present disclosure includes a cartridge that accommodates an examination strip on which a specimen is spread and an examination region for capturing a test substance contained in the specimen is provided, and an accessory that is used for carrying out at least any of collection of the specimen or pretreatment carried out on the specimen before the specimen is spread on the examination strip, where a part of at least any of the examination strip or the accessory includes a portion in which a coloring reaction is exhibited by a reaction with a specimen-derived component different from the test substance in the specimen.

In the immunochromatographic kit according to the present disclosure, the accessory may be a specimen collecting tool that is used for collecting the specimen, and a part of the specimen collecting tool may include the portion that exhibits the coloring reaction.

In the immunochromatographic kit according to the present disclosure, the specimen collecting tool may be a swab, and a part of the swab may include the portion that exhibits the coloring reaction.

In the immunochromatographic kit according to the present disclosure, the accessory may be such that an extraction solution that is used for pretreatment of the specimen and the extraction solution exhibits the coloring reaction.

In the immunochromatographic kit according to the present disclosure, the portion may contain a reagent that exhibits the coloring reaction depending on the pH of the specimen.

In the immunochromatographic kit according to the present disclosure, the portion may contain a reagent that exhibits a coloring reaction by a reaction with a protein as the specimen-derived component.

In the immunochromatographic kit according to the present disclosure, the reagent may be a reagent that exhibits a coloring reaction by a reaction with at least one of a glycoprotein as the protein, albumin, lactoferrin, lysozyme, a CD molecule which is an epithelial cell-derived marker, or a protein constituting a cell skeleton.

In the immunochromatographic kit according to the present disclosure, the reagent may be a reagent that exhibits a coloring reaction by a reaction with mucin, which is a glycoprotein.

In the immunochromatographic kit according to the present disclosure, the specimen may be a nasopharyngeal swab fluid, a nasal cavity swab fluid, nasal discharge, saliva, or a pharyngeal swab fluid.

According to the immunochromatographic kit according to the present disclosure, it is possible to suppress a shortage of an amount of a specimen at the time of collecting the specimen.

### BRIEF DESCRIPTION OF THE DRAWINGS

Fig. 1 is a view showing an overall configuration of an immunochromatographic kit according to a first embodiment.
Fig. 2 is an exploded perspective view of a cartridge.
Fig. 3 is a view showing a reaction step in an examination strip.
Fig. 4 is a view showing the overall configuration of an immunochromatographic kit according to a second embodiment.

### DESCRIPTION OF THE PREFERRED EMBODIMENTS

Hereinafter, specific embodiments of the immunochromatographic kit and the examination apparatus according to the present disclosure will be described with reference to the drawings. The constitutional elements indicated by the same reference numerals in the drawings mean the same constitutional elements.

### "First embodiment"

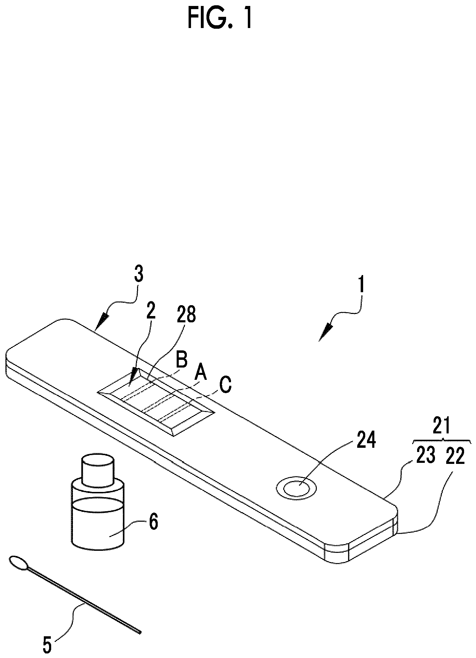

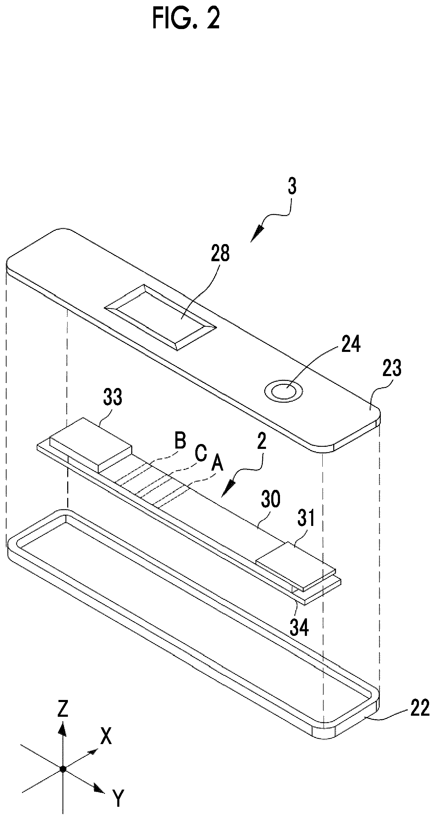

Fig. 1 is a view showing an overall configuration of an immunochromatographic kit 1 according to a first embodiment. Fig. 2 is an exploded perspective view of a cartridge 3 included in the immunochromatographic kit 1.

As shown in Fig. 1 and Fig. 2, the immunochromatographic kit 1 according to the first embodiment includes the cartridge 3 accommodating an examination strip 2, and a specimen collecting tool 5 and an extraction solution 6 as accessories.

The cartridge 3 belongs to a single-use type that is used one by one for each specimen, and it has a case 21 having an elongated rectangular parallelepiped shape. The case 21 is composed of a case main body 22 and a cover member 23. The case main body 22 has an elongated plate shape and includes a recessed part that accommodates the examination strip 2. A cover member 23 includes a specimen dropping port 24 and an observation window 28. It is noted that both the X direction and the Y direction in Fig. 2 are directions along the horizontal plane and are orthogonal to each other. The X direction is a direction along a short side of the case 21 and the Y direction is a direction along a long side of the case 21. The Z direction is a direction along the vertical direction and is orthogonal to the X direction and the Y direction.

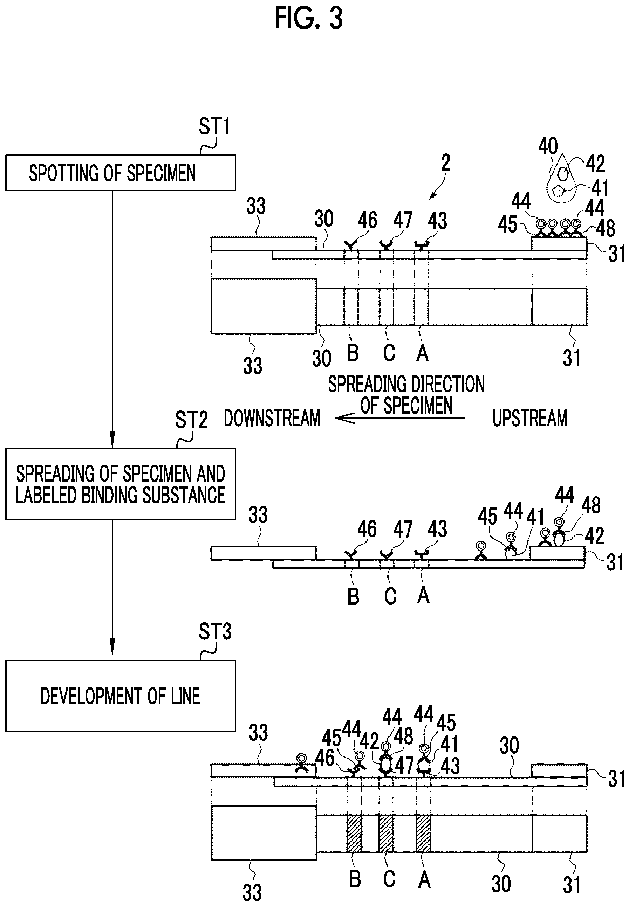

The specimen dropping port 24 is a round hole to which a specimen solution 40 (see Fig. 3) containing a specimen is dropped. The specimen may contain a test substance 41, and in the present embodiment, the specimen is a biological specimen, such as a nasopharyngeal swab fluid, a nasal cavity swab fluid, nasal discharge, saliva, or a pharyngeal swab fluid, which is, on the whole, colorless and transparent. Examples of the test substance 41 include an antigen, an antibody, a protein, and a low-molecular-weight compound.

The observation window 28 is a rectangular opening for observing an examination region A of the examination strip 2 from the outside. A first control region B and a second control region C, which will be described later, can also be observed from the observation window 28 from the outside.

The specimen collecting tool 5 is an accessory for collecting a specimen, and in the present example, it is a swab for collecting a nasopharyngeal swab fluid, a nasal cavity swab fluid, or a nasopharyngeal swab fluid.

The extraction solution 6 contains a surfactant, and it is a solution for destroying cells of a specimen to prepare the specimen solution 40 which is to be subjected to examination. The specimen collected with the test specimen collecting tool 5 is mixed in the extraction solution 6 to prepare the specimen solution 40 containing the specimen. That is, the extraction solution 6 is an accessory for carrying out pretreatment on a specimen before the specimen is spread on the examination strip 2. The specimen solution 40 containing a specimen is added dropwise from the specimen dropping port 24. That is, the dropwise addition of the specimen solution 40 means the dropwise addition of the specimen.

The configuration and functions of the examination strip 2 will be described in detail with reference to Fig. 2 and Fig. 3. Fig. 3 is a view schematically illustrating a reaction that occurs on the examination strip 2 from the moment when the specimen solution 40 is added dropwise to the examination strip 2. As shown in Fig. 2, the examination strip 2 has an elongated rectangular plate shape as a whole and has a carrier 30, a label holding pad 31, an absorption pad 33, and a back pressure-sensitive adhesive sheet 34.

The examination strip 2 has an examination region A in which a specimen (here, the specimen solution 40 containing the specimen) is spread and the test substance 41 contained in the specimen solution 40 is captured. In addition, in the present example, the examination strip 2 includes the first control region B and the second control region C in addition to the examination region A.

The examination region A, the first control region B, and the second control region C are formed in the vicinity of the center of the carrier 30 in the longitudinal direction. In a case where a direction from the label holding pad 31 toward the examination region A or the like is set as a spreading direction (see Fig. 3) of the specimen, the examination region A is located upstream of the first control region B in the spreading direction, and the second control region C is located between the examination region A and the first control region B. All of the examination region A, the first control region B, and the second control region C are provided in a line shape perpendicular to the longitudinal direction of the examination strip 2.

As shown in Fig. 3, a binding substance for test substance capture 43 that captures the test substance 41 is immobilized in the examination region A. The binding substance for test substance capture 43 is a binding substance that specifically binds to the test substance 41. For example, in a case where the test substance 41 is an antigen, the binding substance for test substance capture 43 is an antibody against the antigen, and in a case where the test substance 41 is an antibody, the binding substance for test substance capture 43 is an antigen against the antibody. In a case where the test substance 41 is a protein, a low-molecular-weight compound, or the like, the binding substance for test substance capture 43 is an aptamer with respect to the protein, the low-molecular-weight compound, or the like. In a case where the specimen solution 40 that has been spread contains the test substance 41, a line is developed in the examination region A.

The first control region B is a region for checking whether or not the spreading of the specimen solution 40 is correctly carried out. In a case where the specimen solution 40 is correctly spread, a line is developed in the first control region B. A binding substance for label capture 46 that specifically binds to a first labeled binding substance 45 described later, which is contained in the label holding pad 31, is immobilized in the first control region B.

The second control region C is a region for checking whether or not the amount of the specimen is sufficient. The second control region C is a portion in which a coloring reaction is exhibited by a reaction with a specimen-derived component 42 contained in the specimen, which is different from the test substance 41. In a case where the amount of the specimen contained in the specimen solution 40 is sufficient, a line is developed in the second control region C. For example, a binding substance for specimen-derived component capture 47, which specifically binds to the specimen-derived component 42, is immobilized in the second control region C. For example, in a case where the specimen is a nasal cavity swab fluid or nasal discharge, it is possible to check whether or not the amount of the specimen is sufficient by detecting mucin, which is a glycoprotein usually contained in the nasal cavity swab fluid or nasal discharge, as a specimen-derived component. In this case, it suffices that an anti-mucin antibody may be immobilized in the second control region C as the binding substance for specimen-derived component capture 47.

The carrier 30 is formed from a porous insoluble material such as a nitrocellulose membrane.

The label holding pad 31 is attached to the carrier 30 at a position facing the specimen dropping port 24. The specimen solution 40 dropped from the specimen dropping port 24 is spotted on the label holding pad 31. That is, the label holding pad 31 is disposed at a specimen supply position on the carrier 30.

The label holding pad 31 contains a first labeled binding substance 45 labeled with a gold colloid 44. The first labeled binding substance 45 is a binding substance that specifically binds to a test substance. For example, in a case where the test substance 41 is an antigen, the first labeled binding substance 45 is an antibody against the antigen, and in a case where the test substance 41 is an antibody, the first labeled binding substance 45 is a secondary antibody that is capable of binding to the antibody. In a case where the test substance 41 is a protein, a low-molecular-weight compound, or the like, the first labeled binding substance 45 is an aptamer with respect to the protein, the low-molecular-weight compound, or the like.

The binding substance for test substance capture 43 and the first labeled binding substance 45 may be the same or different from each other. For example, in a case where the test substance 41 is an influenza A type virus or a biomarker thereof, it is possible to use an anti-influenza A type monoclonal antibody (manufactured by Medix Biochemica Inc., product name: Anti-influenza A SPT N-5 7307) as the binding substance for test substance capture 43 and the first labeled binding substance 45.

The label holding pad 31 further contains a second labeled binding substance 48 labeled with the gold colloid 44. The second labeled binding substance 48 is a binding substance that specifically binds to the specimen-derived component 42 different from the test substance 41. For example, in a case of detecting mucin as the specimen-derived component 42, it suffices that an anti-mucin antibody is used as the second labeled binding substance 48.

The binding substance for specimen-derived component capture 47 and the second labeled binding substance 48 may be the same or different from each other as long as they are binding substances that specifically bind to the specimen-derived component 42 to be targeted.

The absorption pad 33 is attached to one end on a side opposite to the other end of the carrier 30 to which the label holding pad 31 is attached. The absorption pad 33 is formed from a porous material similarly to the carrier 30. The absorption pad 33 absorbs the specimen solution 40 that has been spread on the carrier 30.

The back pressure-sensitive adhesive sheet 34 is a base material of which the surface is a pressure-sensitive adhesive surface, and the carrier 30 is adhesively fixed to the back pressure-sensitive adhesive sheet 34.

Hereinafter, with reference to Fig. 3, a description will be made on a step of dropwise adding the specimen solution 40 from the specimen dropping port 24 and then causing a line to be developed in the examination region A, the first control region B, or the second control region C. In Fig. 3, the side view and the top view of the examination strip are shown regarding a step ST1 and a step ST3, and the side view of the examination strip is shown regarding a step ST2. Here, a description will be made using, as an example, a case where a specimen contains the test substance 41, that is, a case where the specimen is positive. It is noted that in Fig. 3, the back pressure-sensitive adhesive sheet 34 is not illustrated in the drawing.

First, as shown in the step ST1, the specimen solution 40, that is, the specimen is spotted from the specimen dropping port 24 to the label holding pad 31.

As shown in the step ST2, the test substance 41 in the specimen solution 40 spotted on the label holding pad 31 specifically binds to the first labeled binding substance 45 of the label holding pad 31. In addition, the specimen-derived component (for example, mucin) 42 in the specimen solution 40 specifically binds to the second labeled binding substance 48 of the label holding pad 31. Then, the specimen solution 40 permeates into the carrier 30 from the label holding pad 31 and then is spread, by the capillary action, to the examination region A side which is the downstream side. In association with this, the specimen, the first labeled binding substance 45, and the first labeled binding substance 45 bound to the test substance 41, as well as the second labeled binding substance 48 and the second labeled binding substance 48 bound to the specimen-derived component 42 are spread in the carrier 30 to the downstream side.

As shown in the step ST3, the test substance 41 that has reached the examination region A is captured by the binding substance for test substance capture 43. As a result, the gold colloid 44 is captured by the binding substance for test substance capture 43 of the examination region A through the test substance 41 and the first labeled binding substance 45. Then, the gold colloid 44 is captured in the examination region A, whereby a line is developed in the examination region A.

The first labeled binding substance 45 that has not bound to the test substance 41 is spread to the downstream side without being captured in the examination region A. The first labeled binding substance 45 that has reached the first control region B is captured by the binding substance for label capture 46. As a result, the gold colloid 44 is captured in the first control region B through the first labeled binding substance 45. Then, the gold colloid 44 is captured in the first control region B, whereby a line is developed in the first control region B.

On the other hand, the specimen-derived component 42 that has reached the second control region C is captured by the binding substance for specimen-derived component capture 47. As a result, the gold colloid 44 is captured in the second control region C through the specimen-derived component 42 and the second labeled binding substance 48. Then, the gold colloid 44 is captured in the second control region C, whereby a line is developed in the second control region C.

In this way, in a case where the specimen is positive, the line is developed in each of the examination region A, the first control region B, and the second control region C of the examination strip 2, as shown in the step ST3 of Fig. 3.

It is noted that in a case where the amount of the specimen is appropriate, and the specimen solution 40 is favorably spread, where the case where the specimen is negative, a line is developed in the first control region B and the second control region C; however, a line is not developed in the examination region A.

On the other hand, in a case where a line is not developed in the first control region B even in a case where the specimen solution 40 is added dropwise, it means that the spreading failure of the specimen solution 40 has occurred. In addition, in a case where a line is not developed in the second control region C even in a case where the specimen solution 40 is added dropwise, it means that the specimen is insufficient. Therefore, in a case where a line is not developed in at least one of the first control region B or the second control region C, it means that the examination has not been appropriately carried out.

As described above, the immunochromatographic kit 1 according to the present embodiment includes the cartridge 3 accommodating the examination strip 2 having the examination region A, and the specimen collecting tool 5 and the extraction solution 6 as accessories for carrying out at least any of the collection of the specimen or the pretreatment of the specimen which is carried out before the specimen is spread on the examination strip 2. In addition, the examination strip 2 includes the second control region C as a portion in which a coloring reaction is exhibited by a reaction with a specimen-derived component different from the test substance in the specimen with the specimen-derived component 42 different from the test substance 41 in the specimen. In a case where the second control region C contains a certain amount or more of the specimen-derived component 42, a color development occurs to a visible extent (coloring occurs) in the second control region C, and a line is developed in the second control region C. The amount of the specimen-derived component 42 is proportional to the amount of the specimen. Therefore, in a case where a line is developed in the second control region C, it can be determined that a required amount of the specimen is contained and thus it has been possible to carry out an appropriate examination. That is, it is possible to check whether or not the specimen solution 40 contains an amount of a specimen necessary for the examination, and to determine whether or not the determination result is appropriate. Therefore, it is possible to suppress such an erroneous determination that a specimen is negative even though the specimen is actually positive in a case where a line is not developed in the examination region A due to an insufficient amount of the specimen due to a collection error.

In the above-described embodiment, the second control region C includes the binding substance for specimen-derived component capture 47 that specifically binds to the specimen-derived component 42, and a color development occurs in a case where the gold colloid 44, which is a label, is captured by the binding due to the antibody-antigen reaction. That is, the second control region C is a portion that is colored by capturing of a label using an antibody-antigen reaction. However, the second control region C may be applied with, instead of the binding substance for specimen-derived component capture 47, a reagent that exhibits a coloring reaction depending on the pH of the specimen or a reagent that exhibits a coloring reaction by causing a chemical reaction with a protein as the specimen-derived component 42 in the specimen, where the chemical reaction is different from the antigen-antibody reaction. Here, the coloring reaction refers to a reaction in which a first color changes to a second color different from the first color. The color development from colorless and transparent to colored is also one form of the reaction in which the first color changes to the second color. The reagent that exhibits a coloring reaction by a reaction with the specimen-derived component 42 in the specimen will be described in a second embodiment described later.

In the above-described embodiment, in the examination strip 2, the second control region C is provided between the examination region A and the first control region B. However, the second control region C may be provided between the examination region A and the label holding pad 31 or downstream of the first control region B.

In the above-described embodiment, the description has been made on the case where the specimen is a nasal cavity swab fluid or nasal discharge and the mucin is targeted as the specimen-derived component 42. However, the specimen-derived component 42 is not limited to mucin. In a case where the specimen is a nasal cavity swab fluid or nasal discharge, it suffices that the specimen-derived component 42 is nasal-derived epithelial cells and secretions that are secreted by the nasal-derived epithelial cells. Mucin is one of the secretions, and examples of the other secretions include various cytokines. In addition, examples of the secretions secreted by the nasal-derived epithelial cells include a cluster of differentiation (CD) molecule, which is an epithelial cell-derived marker, and a protein that constitutes a cytoskeleton, such as keratin. It is noted that in a case where the specimen is not a nasal cavity swab fluid or nasal discharge, the specimen-derived component 42 contained in various specimens can be appropriately set. In addition, it suffices that the second control region C includes the binding substance for specimen-derived component capture 47 that is capable of capturing the specimen-derived component 42 which is determined as a detection target.

In the above-described embodiment, a swab is provided as the specimen collecting tool 5. As the specimen collecting tool 5, an appropriate specimen collecting tool can be proved according to the specimen. For example, in a case where the specimen is saliva, a sponge or the like which absorbs and collects saliva may be used.

In the above-described embodiment, the examination strip 2 includes the second control region C in the examination strip 2 as a portion in which a coloring reaction is exhibited by a reaction with the specimen-derived component 42 different from the test substance 41 in the specimen. However, the one that includes a portion in which a coloring reaction is exhibited by a reaction with a specimen-derived component different from the test substance in the specimen with the specimen-derived component 42 different from the test substance 41 in the specimen is not limited to the examination strip 2. It suffices that a portion in which a coloring reaction is exhibited by a reaction with a specimen-derived component different from the test substance in the specimen with the specimen-derived component 42 different from the test substance 41 in the specimen is provided in a part of at least any of the examination strip or the accessory, which is included in the immunochromatographic kit.

### "Second embodiment"

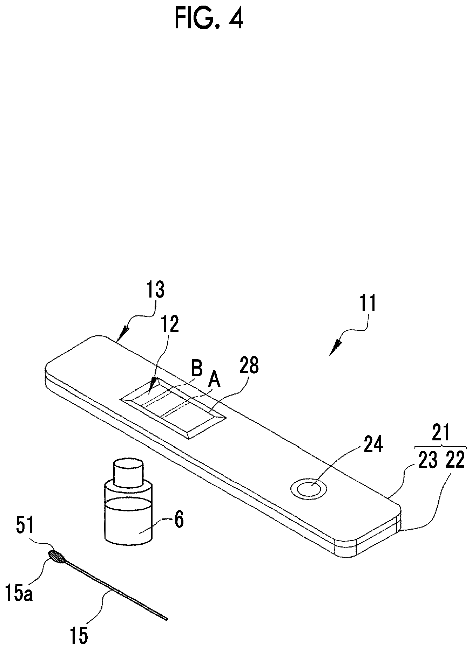

Fig. 4 is a view showing an overall configuration of an immunochromatographic kit 11 according to a second embodiment. As shown in Fig. 4, the immunochromatographic kit 11 according to the second embodiment includes a cartridge 13 accommodating an examination strip 12, and a specimen collecting tool 15 and the extraction solution 6 as accessories.

The cartridge 13 is different from the cartridge 3 of the immunochromatographic kit 1 according to the first embodiment only in that the examination strip 12 does not include the second control region C with respect to the examination strip 2 in the cartridge 3.

As described above, the examination strip 12 includes the examination region A and the first control region B; however, it does not include the second control region C.

On the other hand, the specimen collecting tool 15 that is used for collecting a specimen is a swab for collecting a nasal cavity swab fluid even in the present embodiment; however, it includes a reagent 51 that exhibits a coloring reaction by a reaction with the specimen-derived component 42 different from the test substance 41 in the specimen in a cotton tip 15a which is a part of the swab.

As the reagent 51, a reagent that exhibits a coloring reaction depending on the pH of the specimen or a reagent that exhibits a coloring reaction by a reaction with a protein contained in the specimen is used. The reagent 51 is prepared so that it undergoes a change from the first color to the second color in a case where the test amount of the specimen is equal to or larger than a certain amount or more.

The reagent 51 exhibiting a coloring reaction depending on the pH of the specimen is not particularly limited as long as it is colored by a pH change in a case of being brought into contact with the specimen. For example, in a case where the specimen is a nasal cavity swab fluid or nasal discharge, the pH thereof is 5.5 to 6.5. Therefore, a reagent that undergoes discoloration in a case of being brought into contact with a specimen having a pH of 5.5 to 6.5 is suitable. Examples of the reagent that is discolored by being brought into contact with a specimen having a pH of 5.5 to 6.5 include metacresol purple, 2,4-dinitrophenol, ethyl orange, bromophenol blue, Congo red, alizarin red S, phenol red and cresol red, bromocresol green (BCG), tetrabromophenol blue, and bromocresol purple (BCP). For example, bromophenol blue is light yellow (first color) at a pH of 3.0 and changes to blue (second color) at a pH of 4.6. A solution prepared at pH 3.0, which contains bromophenol blue is used as the reagent 51, and the cotton tip 15a is impregnated with the reagent 51. In a state where the reagent 51 is not contained, the color of the cotton tip 15a having a white color is turned into a light yellow color, which is the first color, by being impregnated with the reagent 51. In a case where a specimen (nasal cavity swab fluid) having a pH of 5.5 to 6.5 is collected in this state, the pH around bromophenol blue approaches the pH of the specimen, the color gradually changes, and then the discoloration occurs to a blue color which is the second color at a pH of 4.6. It suffices that the cotton tip 15a contains an amount of the reagent 51, the amount being prepared so that the pH reaches 4.6 in a case where an amount of a specimen required for the examination is collected.

Examples of the reagent that exhibits a coloring reaction by a reaction with a protein contained in the specimen include reagents that are used in a bisinchoninic acid (BCA) method, a Bradford method, and a Lowry method, which are used for general protein detection. The reagent that is used in the BCA method undergoes a change from the first color which is colorless and transparent to the second color which is in a range of reddish purple to bluish purple, by reacting with overall proteins. The reagent that is used in the Bradford method undergoes a change from the first color which is colorless and transparent to the second color which is blue, by reacting with overall proteins. In addition, the reagent that is used in the Lowry method undergoes a change from the first color which is colorless and transparent to the second color which is blue, by reacting with overall proteins.

In addition, as the reagent that exhibits a coloring reaction by a reaction with a protein contained in the specimen, a color developing reagent such as bromocresol green (BCG), tetrabromophenol blue, or bromocresol purple (BCP) can also be used.

The BCG is colorless and transparent, and it reacts with albumin, transferrin, haptoglobin, ceruloplasmin, complement, C-reactive protein (CRP), a glycoprotein, or the like, thereby undergoing a change from the first color which is colorless and transparent to the second color which is blue. Tetrabromophenol blue is light yellow, and it reacts with albumin, transferrin, haptoglobin, ceruloplasmin, complement, CRP, a glycoprotein, or the like, thereby undergoing a change from the first color which is light yellow to the second color which is blue. In addition, the BCP is colorless and transparent, and it reacts with albumin thereby undergoing a change from the first color which is colorless and transparent to the second color which is bluish purple.

It suffices that a reagent that reacts with a protein, thereby being colored, is appropriately used as the reagent 51, where the protein as a detection target is set according to the kind of specimen.

For example, in a case where the specimen is a nasal cavity swab fluid or nasal discharge, examples of the protein in the specimen include glycoprotein, albumin, immunoglobulin, lactoferrin, and lysozyme. A representative example of the glycoprotein is mucin. Therefore, it suffices that a reagent that exhibits a coloring reaction by a reaction with at least one of glycoprotein, albumin, immunoglobulin, lactoferrin, or lysozyme is used as the reagent 51. In particular, it is preferable to use, as the reagent 51, a reagent that exhibits a coloring reaction by a reaction with mucin, which is a glycoprotein. It is easy to detect mucin since mucus is a main component of a nasal cavity swab fluid, a nasopharyngeal swab fluid, or nasal discharge.

As described above, the immunochromatographic kit 11 according to the present embodiment includes the cartridge 13 accommodating the examination strip 12 having the examination region A, and the specimen collecting tool 15 and the extraction solution 6 as accessories for carrying out at least any of the collection of the specimen or the pretreatment of the specimen which is carried out before the specimen is spread on the examination strip 2. In addition, a part of the specimen collecting tool 15 which is an accessory has a portion (here, the cotton tip 15a) in which a coloring reaction is exhibited by a reaction with the specimen-derived component 42 different from the test substance 41 in the specimen. In the present embodiment, the color of the cotton tip 15a having a light yellow color changes by being brought into contact with a specimen at the time of collecting the specimen. In the present embodiment, it can be determined that a required amount of the specimen is contained in a case where a change occurs from the first color to the second color, for example, from light yellow to blue. That is, it is possible to determine whether positive or negative by using the cartridge 13 after checking whether or not the collection of the amount of the specimen necessary for the examination has been successful. Therefore, it is possible to suppress erroneous determination due to a shortage of an amount of a specimen.

As in the present embodiment, in a case of a form in which the cotton tip 15a of the swab, which is the specimen collecting tool 15, includes a portion in which a coloring reaction is exhibited by a reaction with the specimen-derived component 42, it is possible to check whether or not the collection of the required amount of the specimen has been successful, at the time of collecting the specimen before the specimen collecting tool 15 is inserted into the extraction solution 6 or before the specimen solution 40 containing the specimen is added dropwise to the cartridge 3. As a result, it is possible to respond to a state of a shortage of the amount of the specimen by re-collecting the specimen or the like without using the extraction solution 6 and the cartridge 3.

In the present embodiment, the cotton tip 15a of the swab, which is the specimen collecting tool 15, includes a portion in which a coloring reaction is exhibited by a reaction with a specimen-derived component 42; however, an accessory other than the specimen collecting tool 15 may include a portion in which a coloring reaction is exhibited by a reaction with a specimen-derived component 42. For example, a reagent that exhibits a coloring reaction depending on the pH of the specimen or a reagent that exhibits a coloring reaction by a reaction with a protein contained in the specimen may be added to the extraction solution 6.

The immunochromatographic kit 1 according to the first embodiment and the immunochromatographic kit 11 according to the second embodiment include a portion in which a coloring reaction is exhibited by a reaction with a specimen-derived component different from the test substance in the specimen, in a part of the examination strip and a part of the accessory. Therefore, a user who carries out the examination can easily check visually whether the amount of the specimen is appropriate, which makes it possible to suppress an erroneous determination due to the shortage of the specimen. In particular, it is useful since the occurrence of an erroneous determination due to a shortage of a specimen can be suppressed even in a case where the specimen is collected by a patient himself who is a non-medical worker.

### Examples

Hereinafter, Examples of the technology according to the present disclosed technology will be described. An influenza A type antigen was used as a test substance, and mucin was used as a specimen-derived component for determining the suitability of the amount of the specimen.

### "Example 1"

An examination strip including a control region C that is colored by a reaction with mucin was produced. That is, Example 1 is an example of a form in which a part of the examination strip, which is accommodated in the cartridge of the immunochromatographic kit, includes a portion in which a coloring reaction is exhibited by a reaction with a specimen-derived component different from the test substance in the specimen.

### (1) Examination strip

The configuration and production method for the examination strip used in the present example will be described.

### (Carrier)

A porous carrier (hereinafter, referred to as a carrier) of 60 mm × 300 mm was prepared to form an examination region, a first control region, and a second control region.

### 1) Coating of examination region

A line-shaped examination region was formed along a length direction of 300 mm at a position of 15 mm from one end (hereinafter, referred to as a downstream end) of the carrier in a length direction of 60 mm. An anti-influenza A type monoclonal antibody (Anti-Influenza A SPTN-5 7307, manufactured by Medix Biochemica Inc.) solution prepared to have a concentration of 1.5 mg/mL was applied in a line shape to form an examination region for capturing the influenza A type antigen as a test substance.

### 2) Coating of first control region

A line-shaped first control region was formed along a length direction of 300 mm at a position of 11 mm from one end (hereinafter, referred to as a downstream end) of the carrier in a length direction of 60 mm. An anti-mouse IgG antibody (Anti-mouse IgG (H + L), rabbit F(ab')2, product number: 566-70621, manufactured by FUJIFILM Wako Pure Chemical Corporation) solution prepared to have a concentration of 0.2 mg/mL was applied in a line shape to form a checking region for capturing a first labeled binding substance described later.

### 3) Coating of second control region

A line-shaped second control region was formed along a length direction of 300 mm at a position of 13 mm from a downstream end of the carrier in a length direction of 60 mm. An anti-mucin monoclonal antibody (HIK1083, manufactured by Kanto Chemical Co., Inc.) solution prepared to have a concentration of 1.5 mg/mL was applied in a line shape to form a second control region for capturing mucin as a specimen-derived component.

### 4) Immobilization treatment

After applying the respective solutions to the examination region, the first control region, and the second control region, the carrier was dried at 50°C for 30 minutes using a warm air dryer. After the drying was completed, the carrier was inserted into a vat containing 500 mL of a borate buffer (pH 8.5) of 50 mmol/L, which contained a blocking solution (0.5% by mass casein (derived from milk, product number: 030-01505, manufactured by FUJIFILM Wako Pure Chemical Corporation)), and the carrier was immersed in the borate buffer and then allowed to stand for 30 minutes as it was. Then, the carrier was taken out from the borate buffer, immersed in 500 mL of a washing and stabilizing solution (a 50 mmol/L Tris-HCl (pH 7.5) buffer containing 0.5% by mass sucrose and 0.05% by mass sodium cholate) prepared in another vat, and then allowed to stand as it was for 30 minutes. Thereafter, the carrier was taken out from the Tris-HCl buffer and dried in an environment of 25°C for 24 hours.

Through the step described above, a carrier including the examination region, the first control region, and the second control region was obtained.

### (Label holding pad)

A label holding pad that held a labeled binding substance was produced.

### 1) Production of influenza antibody-modified gold colloid

1 mL of a 50 mmol/L KH₂PO₄ buffer (pH: 7.5) was added to 9 mL of a solution containing a gold colloid having a diameter of 50 nm (product number: EM. GC50, manufactured by BBI Solutions Inc.) to adjust the pH. Thereafter, 1 mL of a solution containing 160 µg/mL of an anti-influenza A type monoclonal antibody (Anti-Influenza A SPTN-5 7307, manufactured by Medix Biochemica Inc.) was added to this solution, and stirring was carried out for 10 minutes. Thereafter, after the resultant mixture was allowed to stand for 10 minutes, 550 µL of an aqueous solution containing 1% by mass polyethylene glycol (PEG; weight-average molecular weight (Mw.): 20,000, product number: 168-11285, manufactured by FUJIFILM Wako Pure Chemical Corporation) was added thereto, followed by stirring for 10 minutes. Subsequently, 1.1 mL of an aqueous solution of 10% by mass bovine serum albumin (BSA; Fraction V, Product No.: A-7906, manufactured by Sigma-Aldrich Co. LLC.) was added thereto, followed by stirring for 10 minutes. This solution was subjected to centrifugal separation for 30 minutes under the conditions of 8,000 × g at 4°C using a centrifugal separator (himac CF16RX, manufactured by Hitachi, Ltd.). The supernatant solution was removed with 1 mL thereof remaining at the bottom of a container, and the gold colloids contained in the 1 mL solution remaining at the bottom of the container were re-dispersed with an ultrasonic washer. Thereafter, 1 mL of the solution was dispersed in 20 mL of a gold colloid preservative solution (a 20 mmol/L Tris-hydrochloric acid (Tris-HCl) buffer (pH: 8.2), 0.05% PEG (Mw.: 20,000), 150 mmol/L NaCl, 1% BSA). Again, centrifugal separation was carried out under the same conditions using the same centrifugal separator, the supernatant solution was removed, the remaining solution containing the gold colloid was subjected to the dispersion of the gold colloid with an ultrasonic washer, and further, the dispersion was carried out in a gold colloid preservative solution to obtain an antibody-modified gold colloid solution.

### 2) Mucin antibody-modified gold colloid

Using an anti-mucin monoclonal antibody (manufactured by NICHIREI BIOSCIENCES INC.) instead of the anti-influenza A type monoclonal antibody, a mucin antibody-modified gold colloid was produced in the same manner as in 1).

### 3) Production of gold colloid holding pad as label holding pad

The anti-influenza A type antibody-modified gold colloid and the mucin antibody-modified gold colloid, which had been respectively produced in 1) and 2), were diluted with water so that a concentration of a Tris-HCl buffer (pH 8.2) was 20 mmol/L, a concentration of PEG (Mw: 20,000) was 0.05% by mass, a concentration of sucrose was 5% by mass, and an optical density of the gold colloid at 520 nm was 0.1 in a case where an optical path length was set to 10 mm, and it was used as a gold colloid coating liquid.

Each glass fiber pad (Glass Fiber Conjugate Pad, manufactured by Millipore Corporation) cut into a size of 12 mm × 300 mm was uniformly coated with 0.8 mL of each of these coating liquid and dried under reduced pressure for 24 hours, thereby obtaining a gold colloid holding pad.

### (Back pressure-sensitive adhesive sheet)

A pressure-sensitive adhesive sheet (manufactured by NIPPN Techno Cluster, Inc.) having a size of 60 mm × 300 nm was prepared as a back pressure-sensitive adhesive sheet.

### (Liquid feeding pad)

A glass fiber pad (Glass Fiber Conjugate Pad, manufactured by Millipore Corporation) cut into a size of 25 mm × 300 mm was used as a liquid feeding pad.

### (Absorption pad)

A glass fiber pad (glass filter paper, manufactured by Advantech Co., Ltd.) cut into a length of 12 mm and a width of 10 mm was used as an absorption pad.

### [Production of examination strip]

Using the above components, an examination strip was produced according to the following procedure.

The carrier on which the examination region had been formed was attached to the back pressure-sensitive adhesive sheet. Next, a double-sided tape (Nitto Denko Corporation) having a width of 3 mm was fixed at a position of 26 mm from the downstream end of the carrier in the direction of 60 mm. Then, the label holding pad was fixed to the carrier so that the downstream end of the double-sided tape and the downstream end of the label holding pad overlapped with each other. The liquid feeding pad was attached to the upstream side of the carrier opposite to the downstream end so that the liquid feeding pad and the carrier overlapped with each other by 7 mm. The sheet-shaped member produced in this way was cut parallel to the length direction of 60 mm to have a width of 5 mm with a guillotine type cutter (CM4000, manufactured by NIPPN Techno Cluster, Inc.). In this way, 60 strips having a width of 5 mm and a length of 60 mm were obtained. One absorption pad was disposed at the downstream end of one strip to be overlapped with each other, thereby obtaining an examination strip.

### (2) Extraction solution

As an extraction solution, 1% BSA-containing phosphate-buffered saline (PBS) was prepared.

### (3) Preparation of specimen solution

Specimen solution 1: The following solution A and solution B were mixed at a ratio of 1:1 to prepare a specimen solution 1 containing an influenza antigen as a test substance and mucin as a specimen-derived component.

Specimen solution 2: The following solution A containing an influenza antigen as a test substance was used as a specimen solution 2. That is, the specimen solution 2 does not contain mucin.
Solution A: A solution obtained by diluting a simulated positive specimen (BD Flu Examen Control A + B -(manufactured by Becton, Dickinson and Company)) 2,560 times by using the extraction solution in (2)
Solution B: A solution obtained by diluting mucin (manufactured by Sigma-Aldrich Co., LLC) 1,000 times by using the extraction solution in (2)

### (4) Evaluation

The specimen solution 1 was spotted on the label holding pad of one examination strip produced in (1), and the specimen solution 1 was spread on the examination strip. As a result, a line was developed in the checking region for the specimen-derived component, in addition to the examination region and the checking region, where the checking region is for indicating the presence or absence of mucin.

The specimen solution 2 was spotted on the label holding pad of the other examination strip produced in (1), and the specimen solution 2 was spread on the examination strip. As a result, a line was developed in the examination region and the checking region. On the other hand, a line was not developed in the checking region for the specimen-derived component, where the checking region is for indicating the presence or absence of mucin.

As described above, by using the examination strip including the checking region for the specimen-derived component, it was possible to detect mucin, which is a specimen-derived component. The development of the line in the checking region for the specimen-derived component means that a certain amount or more of the specimen is contained.

### "Example 2"

A pH indicator was applied onto a cotton tip of a swab for collecting a specimen, where the swab was an accessory. That is, Example 2 is an example of a form in which a part of the accessory of the immunochromatographic kit includes a portion in which a coloring reaction is exhibited by a reaction with a specimen-derived component different from the test substance in the specimen, and it is an example including a reagent that exhibits a coloring reaction depending on the pH of the specimen.

### (1) Preparation of swab

46.5 mL of 0.1 mol/L citric acid was mixed with 3.5 mL of 0.1 mol/L sodium citrate to produce an aqueous citric acid solution having a pH of 3. 0.1 g of bromophenol blue (manufactured by FUJIFILM Wako Pure Chemical Corporation) was dissolved in 50 mL of 95% ethanol (manufactured by FUJIFILM Wako Pure Chemical Corporation), and then 50 mL of an aqueous citric acid solution having a pH of 3 was added thereto to prepare 100 mL of a pH indicator solution (pH: 2.9). A cotton tip of a sterile cotton swab (manufactured by NIPRO CORPORATION) was immersed in the pH indicator solution and then dried in a drying box. As a result, the cotton tip of the cotton swab was light yellow. In this way, a cotton swab (swab) with the pH indicator, which included, at the cotton tip, a portion in which a coloring reaction is exhibited by a reaction with a specimen-derived component, was produced.

### (2) Collection of specimen

A subject was asked to blow the nose using a nose-blowing paper which is a sheet for collecting a specimen, whereby nasal discharge was collected as a specimen. The cotton swab with the pH indicator was rubbed on a nose-blowing paper to adhere nasal discharge to the cotton swab with the pH reagent. In this case, it was confirmed that the cotton tip of the cotton swab with the pH indicator changed from light yellow to blue. The pH of the nasal discharge is about 5.5 to 6.5. Bromophenol blue exhibits a yellow color at a pH of 3 and exhibits a bluish purple color at a pH of 4.6. A case of a state in which the nasal discharge is adhered to the cotton swab with the pH indicator and the pH changes to exhibit a blue color means that the collection of a sufficient amount of the specimen has been successful.

### (3) Preparation of specimen solution

The cotton swab with which the nasal discharge had been collected was immersed in an extraction solution (containing 1% BSA-PBS) in an extraction container and pressed to sandwich the cotton tip part from the outside of the extraction container, and the extraction container was rotated about 10 times. Then, the cotton swab was taken out to obtain a specimen solution for evaluation.

### (4) Evaluation

As a result of spotting the specimen solution on the label holding pad of the examination strip produced in the same manner as in Example 1, thereby spreading the specimen solution on the examination strip, a line was developed in the checking region for the specimen-derived component, where the checking region is for indicating the presence or absence of mucin. As a result, it was possible to confirm that the specimen was collected correctly.

### "Example 3"

A protein coloring reagent was applied onto a cotton tip of a swab for collecting a specimen, where the swab was an accessory. That is, Example 3 is an example of a form in which a part of the accessory of the immunochromatographic kit includes a portion in which a coloring reaction is exhibited by a reaction with a specimen-derived component different from the test substance in the specimen, and it is an example including a reagent that exhibits a coloring reaction by a reaction with a protein as the specimen-derived component.

### (1) Preparation of swab

46.5 mL of 0.1 mol/L citric acid was mixed with 3.5 mL of 0.1 mol/L sodium citrate to produce an aqueous citric acid solution having a pH of 3. 0.1 g of a tetrabromophenol blue acid anhydride (FUJIFILM Wako Pure Chemical Corporation) was dissolved in 10 mL of the aqueous citric acid solution to prepare a protein coloring solution. The protein coloring solution was immersed in a sterile cotton swab (NIPRO CORPORATION) and dried in a drying box. As a result, the cotton tip of the cotton swab was light yellow. In this way, a cotton swab (swab) with the protein coloring reagent, which included, at the cotton tip, a portion in which a coloring reaction is exhibited by a reaction with a specimen-derived component, was produced.

### (2) Collection of specimen

A subject was asked to blow the nose using a nose-blowing paper which is a sheet for collecting a specimen, whereby nasal discharge was collected as a specimen. The cotton swab with the protein coloring reagent was rubbed on a nose-blowing paper to adhere nasal discharge to the cotton swab with the protein coloring reagent. In this case, it was confirmed that the cotton tip of the cotton swab with the protein coloring reagent changed from light yellow to blue. Tetrabromophenol blue forms a composite body with a protein, thereby exhibiting a blue color. A case of a state in which the nasal discharge is adhered to the cotton swab with the protein coloring reagent and then exhibits a blue color means that the collection of the sufficient amount of the specimen has been successful.

### (3) Preparation of specimen solution

The cotton swab with which the nasal discharge had been collected was immersed in an extraction solution (containing 1% BSA-PBS) in an extraction container and pressed to sandwich the cotton tip part from the outside of the extraction container, and the extraction container was rotated about 10 times. Then, the cotton swab was taken out to obtain a specimen solution for evaluation.

### (4) Evaluation

As a result of spotting the specimen solution on the label holding pad of the examination strip produced in the same manner as in Example 1, thereby spreading the specimen solution on the examination strip, a line was developed in the checking region for the specimen-derived component, where the checking region is for indicating the presence or absence of mucin. As a result, it was possible to confirm that the specimen was collected correctly.

The disclosure of JP2022-030380 filed on February 28, 2022 is incorporated in the present specification by reference in its entirety.

All documents, patent applications, and technical standards described in the present specification are incorporated herein by reference, to the same extent as in the case where each of the documents, patent applications, and technical standards is specifically and individually described.

## Claims

1. An immunochromatographic kit comprising:
a cartridge that accommodates an examination strip on which a specimen is spread and an examination region for capturing a test substance contained in the specimen is provided; and
an accessory that is used for carrying out at least any of collection of the specimen or pretreatment carried out on the specimen before the specimen is spread on the examination strip,
wherein a part of at least any of the examination strip or the accessory includes a portion in which a coloring reaction is exhibited by a reaction with a specimen-derived component different from the test substance in the specimen.

2. The immunochromatographic kit according to claim 1,
wherein the accessory is a specimen collecting tool that is used for collecting the specimen, and
a part of the specimen collecting tool includes the portion that exhibits the coloring reaction.

3. The immunochromatographic kit according to claim 2,
wherein the specimen collecting tool is a swab, and
a part of the swab includes the portion that exhibits the coloring reaction.

4. The immunochromatographic kit according to claim 1,
wherein the accessory is an extraction solution that is used for the pretreatment of the specimen, and
the extraction solution exhibits the coloring reaction.

5. The immunochromatographic kit according to any one of claims 1 to 4,
wherein the portion contains a reagent that exhibits the coloring reaction depending on a pH of the specimen.

6. The immunochromatographic kit according to any one of claims 1 to 4,
wherein the portion contains a reagent that reacts with a protein as the specimen-derived component, to exhibit the coloring reaction.

7. The immunochromatographic kit according to claim 6,
wherein the reagent is a reagent that reacts with at least one of a glycoprotein as the protein, albumin, immunoglobulin, lactoferrin, lysozyme, a CD molecule which is an epithelial cell-derived marker, or a protein constituting a cell skeleton, to exhibit the coloring reaction.

8. The immunochromatographic kit according to claim 7,
wherein the reagent is a reagent that reacts with mucin which is the glycoprotein, to exhibit the coloring reaction.

9. The immunochromatographic kit according to any one of claims 1 to 8,
wherein the specimen is a nasopharyngeal swab fluid, a nasal cavity swab fluid, nasal discharge, saliva, or a pharyngeal swab fluid.
